(19) Europäisches Patentamt
European Patent Office
Office européen des brevets

(11) EP 0 897 389 B1

(12) # FASCICULE DE BREVET EUROPEEN

(45) Date de publication et mention de la délivrance du brevet:
**03.07.2002 Bulletin 2002/27**

(21) Numéro de dépôt: **97914363.3**

(22) Date de dépôt: **10.03.1997**

(51) Int Cl.[7]: **C07F 15/00**, A61K 31/28

(86) Numéro de dépôt international:
**PCT/FR97/00419**

(87) Numéro de publication internationale:
**WO 97/33894 (18.09.1997 Gazette 1997/40)**

(54) **COMPLEXES DE PLATINE DINUCLEAIRES, PROCEDE POUR LEUR PREPARATION ET COMPOSITIONS PHARMACEUTIQUES EN CONTENANT**

BINUKLEARE PLATINKOMPLEXE, VERFAHREN ZU IHRER HERSTELLUNG UND PHARMAZEUTISCHE ZUSAMMENSETZUNGEN DIE DIESE KOMPLEXE ENTHALTEN

BINUCLEAR PLATINUM COMPLEXES, METHOD FOR PREPARING SAME AND PHARMACEUTICAL COMPOSITIONS CONTAINING SAID COMPLEXES

(84) Etats contractants désignés:
**DE FR GB**

(30) Priorité: **11.03.1996 FR 9603017**

(43) Date de publication de la demande:
**24.02.1999 Bulletin 1999/08**

(73) Titulaire: **Kidani, Yoshinori**
**Fujisawa-shi, Kanagawa (JP)**

(72) Inventeurs:
- **KIDANI, Yoshinori**
  **Fujisawa 251 (JP)**
- **MAUVERNAY, Rolland-Yves, Debiopharm S.A.**
  **CH-1003 Lausanne (CH)**
- **IRIE, Teruko**
  **Meguro-ku, Tokyo 153 (JP)**

(74) Mandataire: **Boulinguiez, Didier**
**Cabinet Plasseraud**
**84, rue d'Amsterdam**
**75009 Paris (FR)**

(56) Documents cités:
**EP-A- 0 137 900**
**EP-A- 0 214 862**
**EP-A- 0 407 603**
**WO-A-91/03482**
**FR-A- 2 541 284**
**US-A- 4 101 565**
**US-A- 4 283 342**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 897 389 B1

Printed by Jouve, 75001 PARIS (FR)

## Description

La présente invention a pour objet une nouvelle classe de complexes de platine dinucléaires. Elle a également pour objet un procédé pour leur préparation. L'invention concerne aussi les compositions pharmaceutiques les contenant et leur utilisation pour le traitement thérapeutique de divers cancers.

Le cisplatine et l'oxaliplatine sont des agents antinéoplasiques cytostatiques connus, que l'on utilise avantageusement dans le traitement thérapeutique de divers cancers.

Le cisplatine est notamment prescrit pour le traitement de cancers des testicules, des ovaires, de cancers épidermoïdes, pour le traitement des tumeurs au niveau de la tête, du cou, etc.

L'oxaliplatine est prescrit pour le traitement de cancers du même type, principalement le cancer des ovaires, ainsi que le cancer du colon, des voies respiratoires supéricures et les cancers épidermoïdes.

Chacun de ces agents antinéoplasiques possède une spécificité qui lui est propre et, de ce fait, en fonction du stade évolutif de la maladie, de l'état du patient ou du type de cancer à traiter, le praticien peut être amené à prescrire l'un ou l'autre de ces agents. Dans certains cas cependant, l'effet thérapeutique recherché n'est pas atteint ou pour le moins n'atteint pas son niveau maximum.

Tout comme d'autres agents antinéoplasiques cytostatiques, en particulier d'autres dérivés organométalliqucs du platine, le cisplatine et l'oxaliplatine présentent une toxicité intrinsèque qui constitue un facteur limitant sérieux que le praticien doit prendre en compte dans le traitement prescrit. Le cisplatine présente notamment une toxicité rénale ct neuromotrice non négligeable, d'autant plus qu'elle se révèle cumulative. Il provoque également de forts vomissements et peut entraîner une bradycardie, ainsi qu'une myélosuppression. A un degré moindre, l'oxaliplatine entraîne une dysesthésie sensorielle réversible dont on peut aisément modérer les effets en modulant convenablement la dose administrée au patient. Il s'en suit cependant que le praticien se trouve limité sur le plan thérapeutique en termes de moyens de guérison notamment.

L'oxaliplatine appartient à la classe des complexes du platine II de trans-1,2-cyclohexanediamine qui sont actuellement en plein développement. Lesdits complexes ou complexes "dach" font l'objet d'essais cliniques en Europe, notamment en France, ainsi qu'aux Etats-Unis et au Japon et sont particulièrement efficaces contre les mélanomes et les tumeurs d'ovaires, d'utérus, de l'estomac et des intestins, etc.

Le brevet US 4 169 846 décrit de tels complexes, à savoir des complexes de cis-platine (II) et de 1,2-cyclohexanediamine, en particulier le complexe [Pt(II) oxalato (1R, 2R-cyclohexanediamine)] qui est le composé connu sous la dénomination oxaliplatine.

Lesdits complexes "dach", lorsqu'ils sont combinés avec le 5-fluorouracile (5FU), sont particulièrement efficaces contre le cancer de l'estomac.

De plus, lorsqu'ils sont combinés avec le 5-fluorouracile et l'acide folinique, les complexes "dach" sont particulièrement utiles pour le traitement du cancer du colon.

Le brevet EP-A-143 478 concerne une méthode d'administration du cisplatine sous la forme d'une solution aqueuse acide, plus précisément d'acide chlorhydrique présentant un pH compris de préférence entre 3,2 et 3,5 et contenant des ions chlorure par exemple provenant de chlorure de sodium pour stabiliser la solution et éviter ainsi l'hydrolyse.

Le brevet US 4 177 263 qui concerne également le cisplatine décrit une méthode de traitement des tumeurs cancéreuses chez l'animal consistant à injecter par voie parentérale un agent complexe de platine en une quantité efficace pour causer la régression de la tumeur.

Des essais cliniques menés en France sur l'utilisation de la combinaison de l'oxaliplatine et du cisplatine ont montré l'effet remarquable de ladite combinaison sur le cancer du foie. Cependant, on a pu constater l'existence de problèmes de stabilité des complexes associés.

La demande de brevet internationale WO94/12193 décrit notamment une composition destinée à l'administration conjointe de cisplatine et d'oxaliplatine sous la forme d'une combinaison après reconstitution extemporanée par addition de liquide aqueux se présentant sous forme d'un lyophilisat comprenant le cisplatine et l'oxaliplatine dans un rapport pondéral allant de 2:1 à 1:2.

On a maintenant trouvé une nouvelle famille de complexes de platine très stables ayant une activité anticancéreuse.

Les nouveaux complexes de l'invention répondent à l'une des formules générales (I) ou (II) :

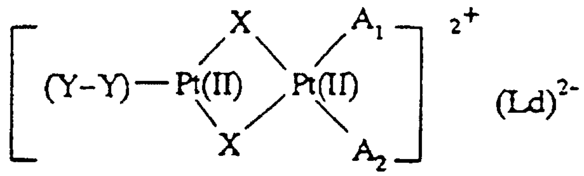

(I)

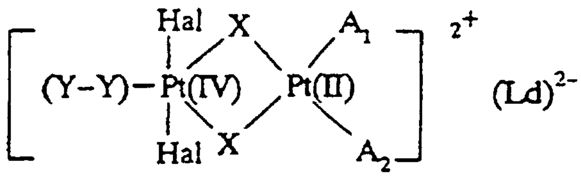

(II)

dans lesquelles :

- Y-Y représente un ligand bidentate amine de formule

  * $NH_2$-$R_1$-$NH_2$ dans laquelle $R_1$ représente un groupe alkylène linéaire ou ramifié en $C_2$-$C_6$ ou un groupe o-phénylène ;
  * ou un groupe de formule

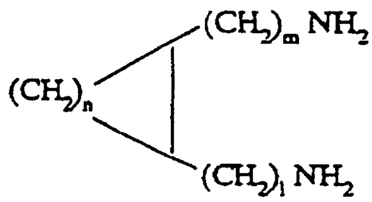

dans laquelle

n = 2, 3, 4, 5
m = 0, 1,
et l = 0,1 ;

- $A_1$ et $A_2$ représentent soit deux ligands monodentates identiques ammine, n-ou isopropylamine ou ($C_3$-$C_7$) cycloalkylamine, soit ils sont liés ensemble et forment un ligand bidentate amine de formule :

  * $NH_2$-$R_1$-$NH_2$ dans laquelle $R_1$ représente un groupe alkylène linéaire ou ramifié en $C_2$-$C_6$ ou un groupe o-phénylène ;
  * ou un groupe de formule

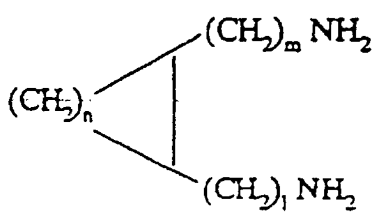

dans laquelle

n = 2, 3, 4, 5

m = 0, 1,
et l = 0, 1 ;

- X représente un ligand de pontage entre les deux platines choisi parmi Cl, Br, F et I ;
- le groupe dissociable Ld représente :

  * soit deux ligands B monodentates choisis parmi $NO_3^-$ ou R $COO^-$, dans lequel R est un résidu d'acide gluconique ou glucuronique ;
  * soit le ligand B-B bidentate choisi parmi

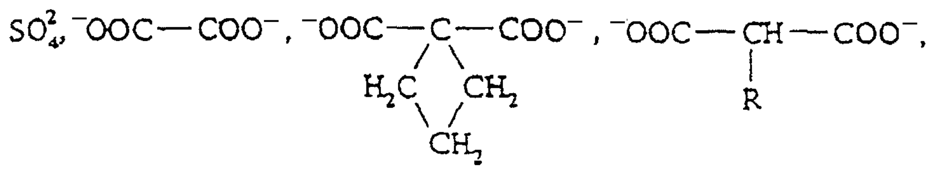

dans lequel R représente l'hydrogène, le groupe méthyle, éthyle, phényle, benzyle ;

- Hal est un atome d'halogène choisi parmi le Cl, Br, F et I.

Une famille de composés préférée de l'invention sont les composés de formule (I) ou (II) dans lesquelles :

- Y-Y représente un ligand bidentate choisi parmi : éthylènediamine, o-phénylènediamine, triméthylènediamine, 1,2-cyclohexanediamine et 2-(aminométhyl) cyclohexylamine ;
- X représente Cl ;
- $A_1$ et $A_2$ représentent soit deux ligands monodentates ammine, soit ils sont liés ensemble et forment un ligand bidentate choisi parmi : éthylènediamine, o-phénylènediamine, triméthylènediamine, 1,2-cyclohexanediamine et 2-(aminométhyl) cyclohexylamine ;
- Ld représente :

  * soit deux ligands B monodentates choisis parmi $NO_3^-$ ou $RCOO^-$, dans lequel R est un résidu d'acide gluconique ou glucuronique ;
  * soit un ligand bidentate B-B choisi parmi $SO_4^{2-}$, ou les dicarboxylates constitués par oxalate, malonate, 1,1-cyclobutanedicarboxylate;

- Hal représente Cl.

L'invention concerne également un procédé pour la préparation des nouveaux complexes de platine dinucléaircs, caractérisé en ce qu'il consiste à faire réagir en solution aqueuse et en proportion équimolaire deux complexes de platine mononucléaires, dont l'un est un complexe de platine di-halogéné C1 et l'autre un complexe de platine C2 comportant soit deux ligands monodentates B soit un ligand bidentate B-B tels que définis ci-dessus, de telle sorte qu'il se forme un pont

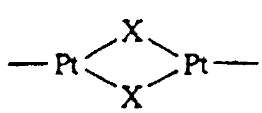

entre les deux complexes, dans lequel X est tel que défini ci-dessus.
Selon un mode préféré de l'invention, le procédé consiste :

a) à dissoudre dans de l'eau distillée le complexe mononucléaire de platine C1 par chauffage au bain-made ;
b) à dissoudre de façon similaire à l'étape a) le complexe mononucléaire de platine C2 dans l'eau distillée ;
c) à mélanger les deux solutions et faire chauffer le mélange réactionnel à 80°C;
d) à abandonner le mélange réactionnel à la température ambiante ; et

e) à filtrer, laver à l'eau tiède, puis à l'alcool le précipité cristallin jaune pâle en forme d'aiguilles ou d'écailles résultant de l'étape d) ;

A l'étape d), on utilise avantageusement comme alcool de l'étbanol.
Des exemples de complexes mononucléaires C1 et C2 sont les complexes de platine mononucléaires suivants :

Complexes C1

- le cisplatine [Pt(II) cis $Cl_2(NH_3)_2$], ci-après "DDP",
- le complexe [Pt(II) $Cl_2$ (1S, 2S-cyclohexanediamine)], ci-après "d-DC dach",
- le complexe [Pt(II) $Cl_2$ (1R, 2R-cyclohexanediamine)], ci-après "l-DC dach",
- le complexe [Pt(II) $Cl_2$ (1R, 2S-cyclohexanediamine)], ci-après "cis-DC dach",
- le complexe [Pt(IV) oxalato trans $Cl_2$ (1R, 2R-cyclohexanediamine)], ci-après "l-OHP.Cl".

Complexes C2

- l'oxaliplatine : [Pt(II) oxalato (1R, 2R-cyclohexanediamine)], ci-après "l-OHP",
- le complexe [Pt(II) oxalato (R, S-cyclohexanediamine)], ci-après "cis-OHP",
- le complexe [Pt(II) $(NO_3)_2$ (1R, 2R-cyclohexanediamine)], ci-après "l-DN dach",
- le complexe [Pt(II) $(NO_3)_2$ (1S, 2S-cyclohexanediamine)], ci-après "d-DN dach",
- le complexe [Pt(II) $(NO_3)_2$ (1R, 2S-cyclohexanediamine)], ci-après "cis-DN dach",
- le carboplatine [Pt(II) cisdiammine (1,1-cyclobutanedicarboxylate)], ci-après "CBDCA",
- le complexe [Pt(IV) oxalato trans $Cl_2$ (1R, 2R-cyclohexanediamine)], ci-après "l-OHP.Cl".

Les complexes de platine mononucléaires C1 et C2 sont avantageusement le cisplatine et l'oxaliplatine.
Les composés de la présente invention de formule (I) sont des principes actifs de compositions pharmaceutiques, dont la toxicité est compatible avec leur utilisation en tant que médicaments.
Ainsi, selon un autre de ses aspects, la présente invention concerne les compositions pharmaceutiques contenant en tant que principe actif un complexe de platine dinucléaire de formule (I) ou (II).
Les composés de la présente invention sont administrés en unité de dosage. Les unités de dosage sont formulées dans des compositions pharmaceutiques dans lesquelles le principe actif est mélangé avec un véhicule pharmaceutique.
Les compositions pharmaceutiques renfermant le complexe du platine dinucléaire de l'invention, en tant que principe actif, peuvent être administrées par voie orale ou parentérale (intramusculaire ou intraveineuse).
Pour une administration par voie orale, les compositions pharmaceutiques peuvent se présenter sous forme de comprimés, gélules, poudres, granulés ou toute autre forme administrable par voie orale. Les compositions pharmaceutiques peuvent en outre contenir des véhicules pharmaceutiquement acceptables compatibles avec les composés de l'invention.
Les compositions pharmaceutiques sont de préférence administrées par voie parentérale (intramusculaire - intraveineuse). Les solutions injectables sont des solutions aqueuses contenant au moins un tampon acide pharmaceutiquement acceptable exempt d'ions chlorure et une substance neutre faisant office de véhicule.
De préférence, on ajuste le pH de la solution injectable à une valeur comprise de préférence entre 6,8 et 7.
Selon l'invention, on utilise avantageusement à titre de tampon acide des acides minéraux ou organiques et leurs sels alcalins pharmaceutiquement acceptables, exempts d'ions chlorure.
Plus particulièrement, on utilise un acide ou un mélange d'acides organiques et de sels alcalins correspondants, pharmaceutiquement acceptables. A titre d'acide organique, on utilise de préférence un acide aminé dicarboxylique, tel que l'acide aspartique ou glutamique et, à titre de sel alcalin, un sel de lithium, sodium ou potassium correspondant. Comme acide minéral, on peut utiliser par exemple de l'acide acétique ou de l'acide phosphorique.
A titre de tampon acide préférentiel, on utilise l'acide glutamique en présence ou non de glutamate de sodium.
Selon l'invention, la composition comprend également généralement une substance neutre faisant office de véhicule, tel qu'un carbohydrate comme le lactose, le glucose, le mannitol ou le sorbitol ou composés similaires.
Une telle solution peut donc être administrée par voie parentérale, le cas échéant, conjointement à d'autres agents cytostatiques, physico-chimiquement compatibles avec les complexes de platine dinucléaires de l'invention et

conformément aux pratiques ayant cours en thérapie anticancéreuse.

Les composés de la présente invention présentent une activité anti-tumorale contre les tumeurs expérimentales chez la souris, telles que L 1210 et sont donc particulièrement utiles dans la chimiothérapie des tumeurs.

Selon un autre de ses aspects, la présente invention concerne l'utilisation des produits de formule (I) pour la préparation de médicaments destinés à traiter divers cancers.

ETUDE PHARMACOLOGIQUE : ACTIVITE ANTITUMORALE

L'activité anti-tumorale des composés de l'invention a été mise en évidence par le test ci-après.

EXPERIENCE

$10^5$ cellules de leucémie L 1210 de souris en suspension saline ont été injectées par voie intrapéritonéale à des groupes de 6 souris CDF1 au jour 0. Le composé à tester a été administré par voie intrapéritonéale 1, 3 et 5 jours après la transplantation des cellules cancéreuses.

On a constitué également des groupes de contrôle de 6 souris qui n'ont pas reçu les composés à tester.

L'efficacité thérapeutique des composés de l'invention a été mesurée par le rapport T/C %. Ce rapport représente 100 fois le temps moyen de survie du groupe ayant reçu le composé à tester divisé par le temps moyen de survie du groupe de comparaison qui n'a pas reçu le composé à tester.

Les résultats sont présentés dans les tableaux 1 et 2 ci-dessous :

TABLEAU 1

Activité antitumorale du complexe de l'invention : le complexe de platine dinucléaire d'oxaliplatine et de cisplatine (l-OHP.DDP) comparée à l'activité des composés de l'art antérieur : cisplatine (DDP), oxaliplatine (l-OHP) et combinaison oxaliplatine/cisplatine (l-OHP + DDP) contre des cellules cancéreuses L 1210.

| | T/C (%) | | | |
|---|---|---|---|---|
| | Dose (mg/kg) | | | |
| | 12,5 | 6,25 | 3,12 | 1,56 |
| DDP | 78 | 245 | 226 | 124 |
| l-OHP | 308 (4/6) | 253 (1/6) | 211 (1/6) | 158 |
| l-OHP + DDP (1:1) | | 230 (1/6) | 284 (2/6) | 167 |
| l-OHP.DDP | 234 (1/6) | 347 (3/6) | 378 (5/6) | 183 |

Les nombres entre parenthèses représentent le nombre de souris guéries dans un groupe de 6 souris.

TABLEAU 2

**Activité antitumorale du complexe de l'invention (l–OHP.DDP) comparée à l'activité de la combinaison connue l–OHP + DDP contre des cellules cancéreuses L 1210 préalablement traitées avec DDP.**

| | T/C (%) | | |
|---|---|---|---|
| | Dose (mg/kg) | | |
| | 6.25 | 3.12 | 1,56 |
| l–OHP + DDP | 105 | 113 | 103 |
| l–OHP . DDP | 207 (2/6) | 398 (5/6) | 345 (6/6) |

Les nombres entre parenthèses représentent le nombre de souris guéries dans un groupe de 6 souris.

Les résultats présentés dans les tableaux 1 et 2 montrent l'efficacité du complexe de platine dinucléaire de l'invention en tant qu'agent antitumoral. En effet, à une dose efficace de 3,12 mg/kg, le complexe de l'invention a permis la guérison de 5 souris sur 6, contre 2 sur 6 pour la combinaison oxaliplatine /cisplatine et 1 sur 6 pour l'oxaliplatine (Tableau 1), sachant qu'une dose inférieure n'est pas suffisante et qu'une dose plus importante est toxique.

On constate, en outre, que lorsque les cellules de leucémie L 1210 sont préalablement traitées au cisplatine, le complexe de l'invention est très efficace à une dose de 1,56 mg/kg : 6 souris sur 6 sont guéries alors que la combinaison connue n'est pas du tout efficace (tableau 2).

STABILITE

On a vérifié d'autre part la stabilité des complexes de platine dinucléaires de l'invention par chromatographie sur papier.

La stabilité a été en effet évaluée par les valeurs de la caractéristique Rf :

$$Rf = \frac{\text{distance parcourue par le composé}}{\text{distance parcourue par le solvant mobile}}$$

Rf est obtenu par chromatographie sur papier (papier filtre Toyo 2 x 20 cm) à la température ambiante en utilisant comme détecteur du $SnCl_2$.

Les résultats sont présentés dans le Tableau 3 ci-dessous.

Les produits de l'invention sont identifiés ci-après par un numéro de code NCU attribué par les inventeurs.

TABLEAU 3

| NCU | Complexes | Rf Solvant éluant 20 % Méthanol | 20 % Ethanol |
|---|---|---|---|
| 501 | l-OHP.DDP | 0,64 | 0,55 |
| --- | l-OHP.DDP * | 0,64 | 0,55 |
| 502 | d-OHP.DDP | 0,62 | 0,52 |
| 503 | cis-OHP.DDP | 0,72 | 0,70 |
| 504 | l-OHP.l-DC dach | 0,68 | 0,38 |
| 507 | DDP.l-DN dac | 0,69 | 0,64 |
| 510 | L-DN dach.l-DC dach | --- | 0,72 |
| 513 | DDP.CBDCA | 0,69 | 0,59 |
| 514 | l-OHP.Cl.DDP | 0,73 | 0,73 |
| --- | DDP | 0,80 | 0,57 |
| --- | l-OHP | 0,65 | 0,63 |
| --- | d-OHP | 0,67 | 0,60 |
| --- | cis-OHP | 0,70 | 0,65 |
| --- | CBDCA | 0,76 | 0,74 |
| --- | $PtCl_2$(l-dach) | --- | 0,74 |
| --- | $Pt(NO_3)_2$(l-dach) | --- | 0,13 |

* le composé marqué par cet astérisque a subit un chauffage dans du HCl 1N

L'invention va maintenant être illustrée de manière plus précise par les exemples illustratifs et non limitatifs ci-après :

EXEMPLE 1 Synthèse d'un complexe de platine dinucléaire de l-OHP et de cisplatine : [(cis-diammine)(1R,2R-cyclohexanediamine)-μ-dichloro-di-Pt(II)] oxalate ou encore [$(NH_3)_2$Pt (μ-Cl)$_2$ Pt (trans-l-dach)] ox, ci-après l-OHP.DDP : NCU 501 de structure chimique :

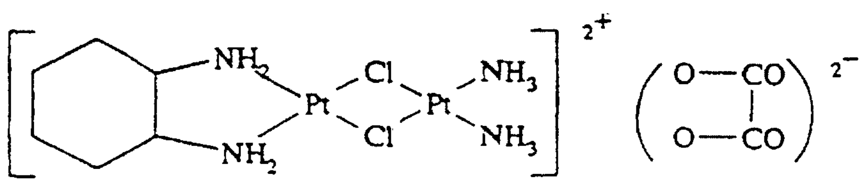

On dissout 0,60 g (2 mmole) de cisplatine (masse molaire = 299 g/mol) dans 90 ml d'eau distillée par chauffage au bain-marie (pH = 5,0). On dissout également 0,80 g (2 mmole) de l-OHP (masse molaire = 397 g/mol) dans 80 ml d'eau distillée en chauffant dans un bain-marie (pH = 5,0). Les deux solutions sont réunies puis on fait chauffer le mélange réactionnel à 80°C. Après 15-30 minutes, on obtient un précipité sous forme d'aiguilles de couleur jaune pâle. On filtre le précipité, lave à l'eau tiède puis à l'alcool. On obtient ainsi 0,61 g du complexe cristallin jaune pâle attendu avec un rendement de 87 %.

Le spectre d'absorption aux infrarouges de ce complexe est représenté sur la figure 1.

| Analyse élémentaire : $C_8H_{20}N_4O_4Pt_2Cl_2$ (masse molaire = 697 g/mol). | | |
|---|---|---|
| | Calculé | Trouvé |
| C | 13,77 % | 13,98 % |
| H | 2,86 % | 3,08 % |
| N | 8,03 % | 8,06 % |
| Pt | 55,95 % | 52,9 % |

EXEMPLE 2 Synthèse d'un complexe de platine dinucléaire de d-OHP et de cisplatine : [(cis-diammine) (1S, 2S-cyclohexanediamine)-μ-dichloro-di-Pt(II)]oxalate ou encore [$(NH_3)_2$Pt (μ-Cl)$_2$ Pt(trans-d-dach)] Ox, ci-après d-OHP. DDP : NCU 502.

On dissout 0,60 g (2 mmole) de cisplatine dans 90 ml d'eau distillée par chauffage au bain-marie (pH = 5,0). On dissout également 0,80 g (2 mmole) de d-OHP dans 80 ml d'eau distillée en chauffant dans un bain-marie (pH = 5,0). Les deux solutions sont réunies puis on fait chauffer le mélange réactionnel à 80°C. Après 15-30 minutes, on obtient un précipité sous forme d'aiguilles de couleur jaune pâle. On filtre le précipité, lave à l'eau tiède puis à l'alcool. On obtient ainsi 0,60 g du complexe cristallin jaune pâle attendu avec un rendement de 86 %.

Le spectre d'absorption aux infrarouges est représenté sur la figure 2.

| Analyse élémentaire : $C_8H_{20}N_4O_4Pt_2Cl_2$ (masse molaire = 697 g/mol). | | |
|---|---|---|
| | Calculé | Trouvé |
| C | 13,77 % | 13,60 % |
| H | 2,86 % | 2,96 % |
| N | 8,03 % | 8,09 % |
| Pt | 55,95 % | --- |

EXEMPLE 3 Synthèse d'un complexe de platine dinucléaire de cis-OHP et de cisplatine : [(cis-diammine) (R, S-cyclohexanediamine)-μ-dichloro-di-Pt(II)]oxalate ou encore [$(NH_3)_2$Pt (μ-Cl)$_2$ Pt(cis-dach)] Ox, ci-après cis-OHP.DDP : NCU 503.

On dissout 0,60 g (2 mmole) de cisplatine dans 90 ml d'eau distillée par chauffage au bain-marie (pH = 5,0). On dissout également 0,80 g (2 mmole) de cis-OHP dans 80 ml d'eau distillée par chauffage au bain-marie (pH = 5,0). Les deux solutions sont réunies et à nouveau chauffées pendant une heure. Après concentration de la solution au 1/3 - 1/2 du volume initial et repos du mélange, on obtient des cristaux en forme d'aiguilles de couleur jaune pâle. On filtre les cristaux, les lave à l'eau tiède puis à l'alcool. On obtient ainsi 0,61 g du complexe cristallin attendu sous la forme d'aiguilles de couleur jaune pâle avec un rendement de 87 %.

Le spectre d'absorption aux infrarouges est représenté sur la figure 3.

| Analyse élémentaire : $C_8H_{20}N_4O_4Pt_2Cl_2$ (masse molaire = 696 g/mol). | | |
|---|---|---|
| | Calculé | Trouvé |
| C | 13,77 % | 13,78 % |
| H | 2,86 % | 2,90 % |
| N | 8,03 % | 8,03 % |
| Pt | 55,95 % | --- |

EXEMPLE 4 Synthèse d'un complexe de platine dinucléaire de l-OHP et de l-DC dach : [bis(1R, 2R-cyclohexanediamine)-μ-dichloro-di-Pt(II)] Oxalate ou encore [(trans-l-dach) Pt (μ-Cl)$_2$Pt(trans-l-dach)] Ox, ci-après l-OHP.l-DC dach : NCU 504.

On dissout 0,40 g (1 mmole) de l-OHP dans 40 ml d'eau distillée par chauffage au bain-marie. On dissout également 0,38 g (1 mmole) de l-DC dach (masse molaire = 379 g/mol) dans 300 ml d'eau distillée par chauffage au bain-marie. Les deux solutions sont réunies puis on fait bouillir le mélange réactionnel pendant environ 1 heure. Après avoir laissé reposer le mélange réactionnel à température ambiante, on obtient des cristaux en forme d'aiguilles de

couleur jaune pâle. Après filtration, les cristaux sont lavés à l'eau tiède, puis à l'alcool donnant 0,30 g du complexe cristallin attendu sous la forme d'aiguilles de couleur jaune pâle avec un rendement de 39 %.

| Analyse élémentaire : $C_{14}H_{28}N_4O_4Pt_2Cl_2$ (masse molaire = 777 g/mol). | | |
|---|---|---|
| | Calculé | Trouvé |
| C | 21,62 % | 20,82 % |
| H | 3,60 % | 3,84 % |
| N | 7,20 % | 7,38 % |
| Pt | 50,19 % | --- |

EXEMPLE 5 Synthèse d'un complexe de platine dinucléaire de l-OHP et de d-DC dach : [(1R, 2R-cyclohexanediamine) (1S, 2S-cyclohexanediamine)-μ-dichloro-di-Pt(II)] Oxalate ou encore [(trans-l-dach) Pt (μ-Cl)$_2$Pt(trans-d-dach)] Ox, ci-après l-OHP.d-DC dach : NCU 505.

On dissout 0,40 g (1 mmole) de l-OHP dans 40 ml d'eau distillée par chauffage au bain-marie et 0,38 g (1 mmole) de d-DC dach (masse molaire = 379 g/mol) dans 300 ml d'eau distillée de façon similaire. Les deux solutions sont réunies puis on fait bouillir le mélange réactionnel pendant environ 1 heure. Après avoir laissé reposer le mélange réactionnel à température ambiante, on obtient des cristaux en forme d'aiguilles de couleur jaune pâle. Après filtration, les cristaux sont lavés à l'eau tiède puis à l'alcool donnant 0,30 g du complexe cristallin avec un rendement de 39 %.

| Analyse élémentaire : $C_{14}H_{28}N_4O_4Pt_2Cl_2$ (masse molaire = 777 g/mol). | | |
|---|---|---|
| | Calculé | Trouvé |
| C | 21,62 % | 21,52 % |
| H | 3,60 % | 3,99 % |
| N | 7,20 % | 7,05 % |
| Pt | 50,19 % | --- |

EXEMPLE 6 Synthèse d'un complexe de platine dinucléaire de l-OHP et de cis-DC dach : [(1R, 2R-cyclohexanediamine) (R, S-cyclohexanediamine)-μ-dichloro-di-Pt(II)] Oxalate ou encore [(trans-l-dach) Pt (μ-Cl)$_2$Pt(cis-dach)] Ox , ci-après l-OHP.cis DC dach NCU 506.

On dissout 0,40 g (1 mmole) de l-OHP dans 40 ml d'eau distillée par chauffage au bain-marie. On dissout également 0,38 g (1 mmole) de cis-DC dach (masse molaire = 379 g/mol) dans 30 ml d'eau distillée de façon similaire. Les deux solutions sont réunies puis on fait bouillir le mélange réactionnel pendant environ 1 heure. Après avoir laissé reposer le mélange réactionnel à température ambiante, on obtient des cristaux en forme d'aiguilles de couleur jaune. On filtre le précipité, lave à l'eau tiède, puis à l'alcool. On obtient ainsi 0,28 g du complexe cristallin attendu sous la forme d'aiguilles de couleur jaune avec un rendement de 36%.

| Analyse élémentaire : $C_{14}H_{28}N_4O_4Pt_2Cl_2$ (masse molaire = 777 g/mol) | | |
|---|---|---|
| | Calculé | Trouvé |
| C | 21,62 % | 20,91 % |
| H | 3,60 % | 3,55 % |
| N | 7,20 % | 7,05 % |
| Pt | 50,19 % | --- |

EXEMPLE 7 Synthèse d'un complexe de platine dinucléaire de cisplatine et de l-DN dach : [(cis-diammine) (1R, 2R-cyclohexanediamine)-μ-dichloro-di-Pt(II)] Nitrate ou encore [$(NH_3)_2$ Pt (μ-Cl)$_2$Pt(trans-l-dach)] $(NO_3)_2$, ci-après DDP. l-DN dach : NCU 507.

On dissout 0,299 g (1 mmole) de cisplatine dans 50 ml d'eau distillée par chauffage au bain-marie (pH environ 5,0). On dissout également 0,433 g (1 mmole) de l-DN dach (masse molaire = 433 g/mol) dans 20 ml d'eau distillée de façon similaire. Les deux solutions sont réunies puis on fait bouillir le mélange réactionnel. Après environ 30 minutes, on obtient un précipité cristallin en forme d'écailles de couleur jaune très pâle. On filtre le précipité, lave à l'eau tiède,

puis à l'alcool. On obtient ainsi 0,30 g du complexe cristallin attendu avec un rendement de 36 %.

| Analyse élémentaire : $C_6H_{20}N_6O_6Pt_2Cl_2$ (masse molaire = 829 g/mol). | | |
|---|---|---|
| | Calculé | Trouvé |
| C | 20,26 % | 19,75 % |
| H | 2,41 % | 2,78 % |
| N | 10,13 % | 10,29 % |
| Pt | 47,04 % | --- |

EXEMPLE 8 Synthèse d'un complexe de platine dinucléaire de cisplatine et de d-DN dach : [(cis-diammine) (1S, 2S-cyclohexanediamine)-μ-dichloro-di-Pt(II)] Nitrate ou encore [$(NH_3)_2$ Pt (μ-Cl)$_2$Pt(trans-d-dach)] $(NO_3)_2$, ci-après DDP. d-DN dach : NCU 508.

On dissout 0,299 g (1 mmole) de cisplatine dans 50 ml d'eau distillée par chauffage au bain-marie (pH environ 5,0). On dissout également 0,433 g (1 mmole) de d-DN dach (masse molaire = 433 g/mol) dans 20 ml d'eau distillée de façon similaire. Les deux solutions sont réunies puis on fait bouillir le mélange réactionnel. Après environ 30 minutes, on obtient un précipité cristallin en forme d'écailles, de couleur jaune très pâle. On filtre le précipité, lave à l'eau tiède, puis à l'alcool. On obtient ainsi 0,28 g du complexe cristallin attendu avec un rendement de 34 %.

| Analyse élémentaire : $C_6H_{20}N_6O_6Pt_2Cl_2$ (masse molaire = 829 g/mol). | | |
|---|---|---|
| | Calculé | Trouvé |
| C | 20,26 % | 20,16 % |
| H | 2,41 % | 2,31 % |
| N | 10,13 % | 10,03 % |
| Pt | 47,04 % | --- |

EXEMPLE 9 Synthèse d'un complexe de platine dinucléaire de cisplatine et de cis-DN dach : [(cis-diammine) (R, S-cyclohexanediamine)-μ-dichloro-di-Pt(II)] Nitrate ou encore [$(NH_3)_2$ Pt (μ-Cl)$_2$Pt(cis-dach)] $(NO_3)_2$, ci-après DDP. cis-DN dach : NCU 509.

On dissout 0,299 g (1 mmole) de cisplatine dans 50 ml d'eau distillée par chauffage au bain-marie (pH environ 5,0). On dissout également 0,433 g (1 mmole) de cis-DN dach (masse molaire = 433 g/mol) dans 20 ml d'eau distillée de façon similaire. Les deux solutions sont réunies puis on fait bouillir le mélange réactionnel. Après environ 30 minutes, on obtient un précipité cristallin en forme d'écailles de couleur jaune très pâle. On filtre le précipité, lave à l'eau tiède, puis à l'alcool. On obtient ainsi 0,28 g du complexe cristallin attendu avec un rendement de 34 %.

| Analyse élémentaire : $C_6H_{20}N_6O_6Pt_2Cl_2$ (masse molaire = 829 g/mol). | | |
|---|---|---|
| | Calculé | Trouvé |
| C | 20,26 % | 20,05 % |
| H | 2,41 % | 2,25 % |
| N | 10,13 % | 10,01 % |
| Pt | 47,04 % | --- |

EXEMPLE 10 Synthèse d'un complexe de platine dinucléaire de l-DC dach et de l-DC dach : [bis (1R, 2R-cyclohexanediamine)-μ-dichloro-di-Pt(II)] Nitrate ou encore [(trans-l-dach) Pt (μ-Cl)$_2$Pt(trans-l-dach)] $(NO_3)_2$, ci-après l-DC dach. l-DN dach : NCU 510.

On dissout 0,38 g (1 mmole) de l-DC dach dans 300 ml d'eau distillée par chauffage au bain-marie (pH environ 5,0). On dissout également 0,433 g (1 mmole) de l-DN dach (masse molaire = 433 g/mol) dans 20 ml d'eau distillée de façon similaire. Les deux solutions sont réunies puis on fait bouillir le mélange réactionncl. Après environ 30 minutes, on obtient un precipité cristallin en forme d'écailles de couleur jaune très pâle. Ces cristaux sont filtrés, lavés à l'eau tiède, puis à l'alcool. On obtient ainsi 0,20 g du complexe cristallin attendu avec un rendement de 25 %.

| Analyse élémentaire : $C_{12}H_{28}N_6O_6Pt_2Cl_2$ (masse molaire = 813 g/mol). | | |
|---|---|---|
| | Calculé | Trouvé |
| C | 17,71 % | 18,05 % |
| H | 3,44 % | 3,40 % |
| N | 10,33 % | 9,79% |
| Pt | 47,37 % | --- |

EXEMPLE 11 Synthèse d'un complexe de platine dinucléaire de d-DC dach et de l-DN dach : [(1R,2R-cyclohexanediamine) (1S,2S-cyclohexanediamine)-μ-dichloro-di-Pt(II)] Nitrate ou encore [(trans-l-dach) Pt ($\mu$-Cl)$_2$Pt(trans-d-dach)] $(NO_3)_2$, ci-après d-DC dach.l-DN dach : NCU 511.

On dissout 0,38 g (1 mmole) de d-DC dach dans 300 ml d'eau distillée par chauffage au bain-marie (pH environ 5,0). On dissout également 0,433 g (1 mmole) de 1-DN dach (masse molaire = 433 g/mol) dans 20 ml d'eau distillée de façon similaire. Les deux solutions sont réunies puis on fait bouillir le mélange réactionnel. Après environ 30 minutes, on obtient un précipité cristallin en forme d'écailles de couleur jaune très pâle. Ces cristaux sont filtrés, lavés à l'eau tiède, puis à l'alcool. On obtient ainsi 0,20 g du complexe cristallin attendu avec un rendement de 25 %.

| Analyse élémentaire : $C_{12}H_{28}N_6O_6Pt_2Cl_2$ (masse molaire = 813 g/mol). | | |
|---|---|---|
| | Calculé | Trouvé |
| C | 17,71 % | 17,65 % |
| H | 3,44 % | 3,34 % |
| N | 10,33 % | 10,30 % |
| Pt | 47,97 % | --- |

EXEMPLE 12 Synthèse d'un complexe de platine dinucléaire de cis-DC dach et de l-DN dach : [(1R,2R-cyclohexanediamine) (R,S-cyclohexane diamine)-μ-dichloro-di-Pt(II)] Nitrate ou encore [(trans-l-dach) Pt ($\mu$-Cl)$_2$Pt(cis-dach)] $(NO_3)_2$, ci-après cis-DC dach.l-DN dach : NCU 512.

On dissout 0,38 g (1 mmole) de cis-DC dach dans 300 ml d'eau distillée par chauffage au bain-marie (pH environ 5,0). On dissout également 0,433 g (1 mmole) de l-DN dach (masse molaire = 433 g/mol) dans 20 ml d'eau distillée de façon similaire. Les deux solutions sont réunies puis on fait bouillir le mélange réactionnel pendant 30 minutes. Après concentration de la solution à 50 % du volume initial, on abandonne le mélange à la température ambiante. On obtient un précipité cristallin en forme d'aiguilles de couleur jaune très pâle. Ces cristaux sont filtrés, lavés à l'eau tiède, puis à l'alcool. On obtient ainsi 0,15 g du complexe cristallin attendu avec un rendement de 18 %.

| Analyse élémentaire : $C_{12}H_{28}N_6O_6Pt_2Cl_2$ (masse molaire = 813 g/mol). | | |
|---|---|---|
| | Calculé | Trouvé |
| C | 17,71 % | 18,01 % |
| H | 3,44 % | 3,45 % |
| N | 10,33 % | 10,23 % |
| Pt | 47,97 % | --- |

EXEMPLE 13 Synthèse d'un complexe de platine dinucléaire de DDP et CBDCA : [bis (cis-diammine)-μ-dichloro-di-Pt(II)] 1,1-cyclobutanedicarboxylate, ou encore [$(NH_3)_2$Pt ($\mu$-Cl)$_2$ Pt $(NH_3)_2$] CBDCA, ci-après DDP.CBDCA : NCU 513.

On dissout 0,30 g (1 mmole) de DDP dans 50 ml d'eau distillée en portant à ébullition. On ajoute ensuite 0,37 g (1 mmole) de CBDCA au milieu réactionnel et une goutte de HCl 1N, et on fait bouillir à nouveau le mélange pendant 30 minutes. Après 30 minutes environ, on obtient des cristaux de couleur marron jaunâtre en forme d'aiguilles. On filtre, lave à l'eau chaude, puis à l'alcool. On obtient ainsi 0,50 g du complexe cristallin attendu avec un rendement de 75 %.

Le spectre d'absorption aux infrarouges se trouve à la figure 4.

| Analyse élémentaire $C_6H_{12}N_4O_4Pt_2Cl_2$ (masse molaire = 671 g/mol). | | |
|---|---|---|
| | Calculé | Trouvé |
| C | 10,73 % | 10,18 % |
| H | 2,68 % | 2,14 % |
| N | 8,34 % | 8,33 % |
| Pt | 58,12 % | --- |

EXEMPLE 14 Synthèse d'un complexe de platine dinucléaire de l-OHP.Cl et de DDP : [(cis-diammine) (trans-dichloro) (1R, 2R-cyclohexanediamine)-μ-dichloro-Pt(II)-Pt(IV)] Oxalate ou encore [$(NH_3)_2$Pt(II) (μ-$Cl)_2$ Pt(IV) (trans-$Cl_2$-trans-l-dach)] Ox, ci-après l-OHP.Cl-DDP : NCU 514.

On dissout 0,47 g (1 mmole) de l-OHP.Cl (masse molaire = 469 g/mol) dans 50 ml d'eau distillée en chauffant dans un bain-marie et 0,3 g (1 mmole) de DDP (masse molaire = 299 g/mol) dans 30 ml d'eau distillée de façon similaire. Les deux solutions sont réunies puis on fait bouillir le mélange réactionnel pendant 30 minutes. On obtient des précipités cristallins de couleur jaune très pâle lorsque la réaction est terminée. Les précipités sont filtrés, lavés à l'eau tiède, puis à l'alcool donnant 0,33 g du complexe cristallin attendu avec un rendement de 50 %.

Le spectre d'absorption aux infrarouges se trouve à la figure 5.

| Analysc élémentaire : $C_8H_{20}N_4O_4Cl_4Pt_2$ (masse molaire = 768 g/mol). | | |
|---|---|---|
| | Calculé | Trouvé |
| C | 12,53 % | 12,50 % |
| H | 2,61 % | 2,75 % |
| N | 7,29 % | 7,40 % |
| Pt | 50,78 % | --- |

**Revendications**

1. Composé de formule (I) ou (II):

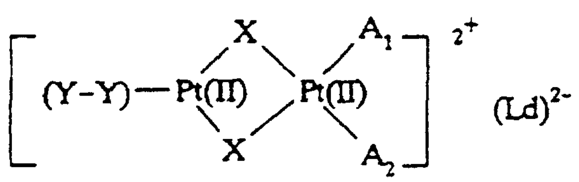

(I)

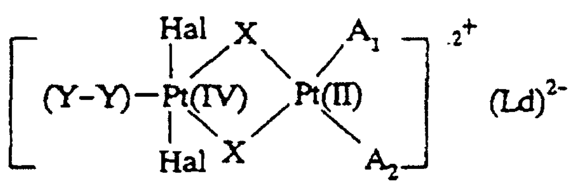

(II)

dans lesquelles :

- Y-Y représente un ligand bidentate amine de formule
  - $NH_2$-$R_1$-$NH_2$ dans laquelle $R_1$ représente un groupe alkylène linéaire ou ramifié en $C_2$-$C_6$ ou un groupe o-phénylène;
  - ou un groupe de formule

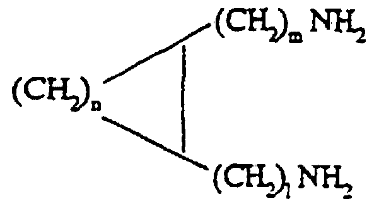

dans laquelle

n = 2, 3, 4, 5
m = 0, 1,
et l = 0, 1 ;

- $A_1$ et $A_2$ représentent soit deux ligands monodentates identiques ammine, n-ou isopropylamine ou ($C_3$-$C_7$) cycloalkylamine , soit ils sont liés ensemble et forment un ligand bidentate amine de formule :

  * $NH_2$-$R_1$-$NH_2$ dans laquelle $R_1$ représente un groupe alkylène linéaire ou ramifié en $C_2$-$C_6$ ou un groupe O-phénylène ;
  * ou un groupe de formule

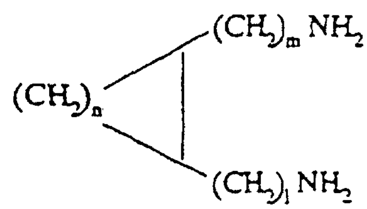

dans laquelle

n = 2, 3, 4, 5
m = 0, 1,
et l = 0, 1 ;

- X représente un ligand de pontage entre les deux platines choisi parmi Cl, Br, F et I ;
- le groupe dissociable Ld représente :

  * soit deux ligands B monodentates choisis parmi $NO_3^-$ ou R $COO^-$, dans lequel R est un résidu d'acide gluconique ou glucuronique ;
  * soit le ligand B-B bidentate choisi parmi

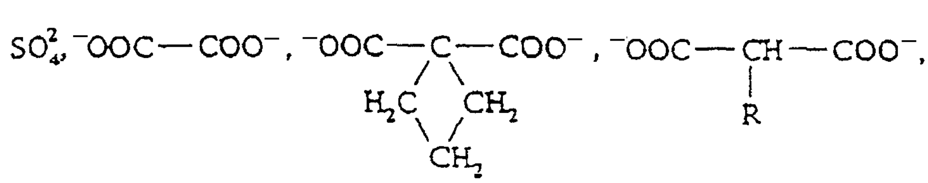

dans lequel R représente l'hydrogène, le groupe méthyle, éthyle, phényle, benzyle ; et

- Hal est un atome d'halogène choisi parmi le Cl, Br, F et I.

**2.** Composé de formule (I) ou (II) selon la revendication 1 **caractérise en ce que** :

- Y-Y représente un ligand bidentate choisi parmi : éthylènediamine, o-phénylènediamine, triméthylènediamine,

1,2-cyclohexanediamine et 2-(aminométhyl) cyclohexylamine ;

- X représente Cl;
- $A_1$ et $A_2$ représentent soit deux ligands monodentates ammine, soit ils sont liés ensemble et forment un ligand bidentate choisi parmi : éthylènediamine, o-phénylènediamine, timéthylènediamine, 1,2-cyclohexanediamine et 2-(aminométhyl) cyclohexylamine ;
- Ld représente :

  * soit deux ligands B monodentates choisis parmi $NO_3^-$ ou $RCOO^-$, dans lequel R est un résidu d'acide gluconique ou glucuronique ;
  * soit un ligand bidentate B-B choisi parmi $SO_4^{2-}$, ou les dicarboxylates constitués par oxalate, malonate, 1,1-cyclobutanedicarboxylate ; et

- Hal représente Cl.

**3.** Composé de formule (I) ou (II) selon la revendication 1 **caractérisé en ce qu'**il est choisi parmi les composés suivants :

[(cis-diammine) (1R, 2R-cyclohexanediamine)-μ-dichloro-di-Pt(II)] oxalate,
[(cis-diammine) (1S, 2S-cyclohexanediamine)-μ-dichloro-di-Pt(II)] oxalate,
[(cis-diammine) (R, S-cyclohexanediamine)-μ-dichloro-di-Pt(II)]oxalate,
[bis(1R, 2R-cyclohexanediamine)-μ-dichloro-di-Pt(II)] Oxalate,
[(1R, 2R-cyclohexanediamine) (1S, 2S-cyclohexanediamine)-μ-dichloro-di-Pt(II)] Oxalate,
[(1R, 2R-cyclohexanediamine) (R, S-cyclohexanediamine)-μ-dichloro-di-Pt(II)] Oxalate
[(cis-diammine) (1R, 2R-cyclohexanediamine)-μ-dichloro-di-Pt(II)] Nitrate,
[(cis-diammine) (1S, 2S-cyclohexanediamine)-μ-dichloro-di-Pt(II)] Nitrate,
[(cis-diammine)(R,S-cyclohexanediamine)-μ-dichloro-di-Pt(II)] Nitrate,
[bis (1R, 2R-cyclohexanediamine)-μ-dichloro-di-Pt(II)] Nitrate,
[(1R, 2R-cyclohexanediamine)(1S, 2S-cyclohexane-diamine)-μ-dichloro-di-Pt(II)]Nitrate.
[(1R, 2R-cyclohexanediamine)(R, S-cyclohexane diamine)-μ-dichloro-di-Pt(II)] Nitrate,
[bis (cis-diammine)-μ-dichloro-di-Pt(II)]1,1-cyclobutanedicarboxylate,
[(cis-diammine) (trans-dichloro) (1R, 2R-cyclohexanediamine)-μ-dichloro-Pt(II)-Pt(IV)] Oxalate,

**4.** Procédé pour la préparation des composés de formule (I) ou (II) **caractérisé en ce qu'**il consiste à faire réagir en solution aqueuse et en proportion équimolaire deux complexes de platine mononucléaires, dont l'un est un complexe de platine di-halogéné C1 et l'autre un complexe de platine C2 comportant soit deux ligands monodentates B soit un ligand bidentate B-B tels que définis dans la revendication 1, de telle sorte qu'il se forme un pont

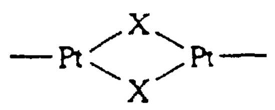

entre les deux complexes, dans lequel X est tel que défini dans la revendication 1.

**5.** Procédé selon la revendication 4, **caractérisé en ce qu'**il consiste :

a) à dissoudre dans de l'eau distillée le complexe mononucléaire de platine C1 par chauffage au bain-marie ;
b) à dissoudre de façon similaire à l'étape a) le complexe mononucléaire de platine C2 dans l'eau distillée ;
c) à mélanger les deux solutions et faire chauffer le mélange réactionnel à 80°C ;
d) à abandonner le mélange réactionnel à la température ambiante ; et
e) à filtrer, laver à l'eau tiède, puis à l'alcool le précipité cristallin jaune pâle en forme d'aiguilles ou d'écailles résultant de l'étape d).

**6.** Procédé selon la revendication 5, **caractérisé en ce que** les complexes C1 sont choisis parmi :

. le cisplatine [Pt(II) cis $Cl_2$ $(NH_3)_2$],
. le complexe [Pt(II) $Cl_2$ (1S, 2S-cyclohexanediamine)],

- le complexe [Pt(II) $Cl_2$ (1R, 2R-cyclohexanediamine)],
- le complexe [Pt(II) $Cl_2$ (1R, 2S-cyclohexanediamine)],
- le complexe [Pt(IV) oxalato trans $Cl_2$ (1R, 2R-cyclohexanediamine)],

et les complexes C2 sont choisis parmi :

- l'oxaliplatine : [Pt(II) oxalato (1R, 2R-cyclohexanediamine)],
- le complexe [Pt(II) oxalato (R, S-cyclohexanediamine)],
- le complexe [Pt(II) $(NO_3)_2$ (1R, 2R-cyclohexanediamine)],
- le complexe [Pt(II)$(NO_3)_2$(1S, 2S-cyclohexanediamine)],
- le complexe [Pt(II)$(NO_3)_2$(1R, 2S-cyclohexanediamine)],
- le carboplatine [Pt(II) cisdiammine (1,1-cyclobutanedicarboxylate)],
- le complexe [Pt(IV) oxalato trans $Cl_2$ (1R, 2R-cyclohexanediamine)].

**7.** Composition pharmaceutique contenant à titre de principe actif un composé selon l'une quelconque des revendications 1 à 3.

## Patentansprüche

**1.** Verbindungen der Formeln (I) oder (II)

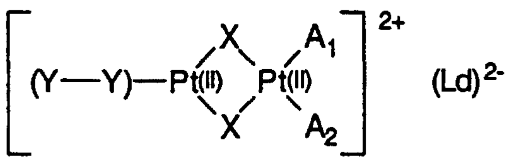

(I)

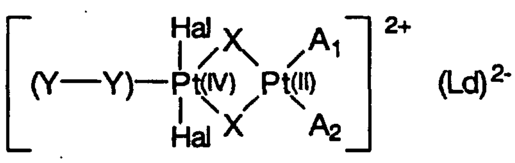

(II)

in welchen

- Y-Y einen zweizähnigen Aminliganden der Formel
  - $NH_2$-$R_1$-$NH_2$, in welchem $R_1$ eine lineare oder verzweigte $C_2$-$C_6$ Alkylengruppe oder eine o-Phenylengruppe ist,
  - oder eine Gruppe der Formel

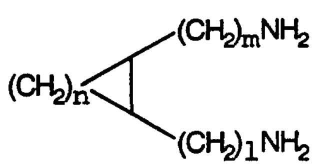

in welcher

n = 2,3,4,5
m = 0,1, und
l = 0,1 ist,

darstellt,

- $A_1$ und $A_2$ entweder zwei identische, einzähnige Ammin-, n- oder Isopropylaminliganden oder ($C_3$-$C_7$)-Cycloalkylaminliganden darstellen, oder miteinander zu einem zweizähnigen Aminliganden der Formel

  • $NH_2$-$R_1$-$NH_2$, in welchem $R_1$ eine lineare oder verzweigte $C_2$-$C_6$ Alkylengruppe oder eine o-Phenylengruppe ist,
  • oder einer Gruppe der Formel

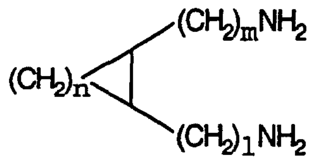

in welcher

n = 2,3,4,5
m = 0,1, und
l = 0,1 ist,

verbunden sind,

- X einen Brückenliganden zwischen den beiden Platinzentren, ausgewählt aus Cl, Br, F und I, darstellt,
- die dissoziierbare Gruppe Ld

  • entweder zwei einzähnige Liganden B, ausgewählt aus $NO_3^-$ und R-$COO^-$, wobei R ein Gluconsäure- oder Glucuronsäurerest ist,
  • oder einen zweizähnigen Liganden B-B, ausgewählt aus

$SO_4^{2-}$, $^-OOC$-$COO^-$, 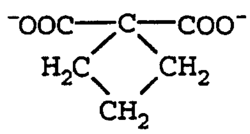 , 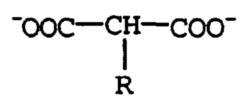

wobei R für Wasserstoff, eine Methyl-, Ethyl-, Phenyl- oder Benzylgruppe steht,

darstellt, und

- Hal ein Halogenatom, ausgewählt aus Cl, Br, F und I, ist.

**2.** Verbindungen der Formeln (I) oder (II) gemäß Anspruch 1, **dadurch gekennzeichnet, dass**

- Y-Y einen zweizähnigen Liganden darstellt, der aus Ethylendiamin, o-Phenylendiamin, Trimethylendiamin, 1,2-Cyclohexandiamin und 2-(Aminomethyl)cyclohexylamin ausgewählt ist,
- X Chlor darstellt,
- $A_1$ und $A_2$ entweder zwei einzähnige Amminliganden darstellen, oder zu einem zweizähnigen Liganden, ausgewählt aus Ethylendiamin, o-Phenylendiamin, Trimethylendiamin, 1,2-Cyclohexandiamin und 2-(Aminomethyl)cyclohexylamin, verbunden sind,
- Ld

  • entweder zwei einzähnige Liganden B, ausgewählt aus $NO_3^-$ oder R-$COO^-$, wobei R ein Gluconsäure- oder Glucuronsäurerest ist,
  • oder einen zweizähnigen Liganden B-B, ausgewählt aus $SO_4^{2-}$ oder den Dicarboxylaten, die aus Oxalat, Malonat und 1,1-Cyclobutandicarboxylat ausgewählt sind,

darstellt, und
- Hal für Cl steht.

3. Verbindungen der Formeln (I) oder (II) gemäß Anspruch 1, **dadurch gekennzeichnet, dass** sie aus den folgenden Verbindungen ausgewählt sind:

[(cis-Diammin)(1R,2R-cyclohexandiamin)-μ-dichlor-di-Pt(II)]oxalat,
[(cis-Diammin)(1S,2S-cyclohexandiamin)-μ-dichlor-di-Pt(II)]oxalat,
[(cis-Diammin)(R,S-cyclohexandiamin)-μ-dichlor-di-Pt(II)]oxalat,
[bis(1R,2R-Cyclohexandiamin)-μ-dichlor-di-Pt(II)] oxalat,
[(1R,2R-Cyclohexandiamin)(1S,2S-cyclohexandiamin)-μdichlor-di-Pt(II)]oxalat,
[(1R,2R-cyclohexandiamin)(R,S-cyclohexandiamin)-μdichlor-di-Pt(II)]oxalat,
[(cis-Diammin)(1R,2R-cyclohexandiamin)-μ-dichlor-di-Pt(II)]nitrat,
[(cis-Diammin)(1S,2S-cyclohexandiamin)-μ-dichlor-di-Pt(II)]nitrat,
[(cis-Diammin)(R,S-cyclohexandiamin)-μ-dichlor-di-Pt(II)]nitrat,
[bis(1R,2R-Cyclohexandiamin)-μ-dichlor-di-Pt(II)] nitrat,
[(1R,2R-Cyclohexandiamin)(1S,2S-cyclohexandiamin)-μdichlor-di-Pt(II)]nitrat,
[(1R,2R-Cyclohexandiamin)(R,S-cyclohexandiamin)-μdichlor-di-Pt(II)]nitrat,
[bis(cis-Diammin)-μ-dichlor-di-Pt(II)]1,1-cyclobutandicarboxylat, und
[(cis-Diammin)(trans-dichlor)(1R,2Rcyclohexandiamin)-μ-dichlor-Pt(II)-Pt(IV)]oxalat.

4. Verfahren zur Herstellung der Verbindungen der Formeln (I) oder (II), **dadurch gekennzeichnet, dass** zwei einkernige Platinkomplexe, von denen einer ein Platin-dihalogenkomplex C1 und der andere ein Platinkomplex C2 ist, der entweder zwei einzähnige Liganden B oder einen zweizähnigen Liganden B-B gemäß Anspruch 1 enthält, in wässriger Lösung und in äquimolaren Mengen derart umgesetzt werden, dass eine Brücke

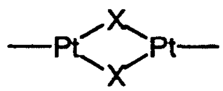

zwischen den beiden Komplexen gebildet wird, in der X gemäß Anspruch 1 definiert ist.

5. Verfahren gemäß Anspruch 4, **dadurch gekennzeichnet, dass**

a) der einkernige Platinkomplex C1 unter Erwärmung im Wasserbad in destilliertem Wasser aufgelöst wird,
b) auf gleiche Weise wie in Schritt a) der einkernige Platinkomplex C2 in destilliertem Wasser aufgelöst wird,
c) die beiden Lösungen vermischt werden und die Reaktionsmischung auf 80°C erhitzt wird,
d) die Reaktionsmischung auf Umgebungstemperatur abgekühlt wird, und
e) als Ergebnis des Schrittes d) der blassgelbe kristalline Niederschlag in Form von Nadeln oder Schuppen filtriert und mit lauwarmem Wasser und anschließend mit Alkohol gewaschen wird.

6. Verfahren nach Anspruch 5, **dadurch gekennzeichnet, dass** die Komplexe C1 ausgewählt sind aus

- Cisplatin [Pt(II)cis-$Cl_2(NH_3)_2$],
- dem Komplex [Pt(II)$Cl_2$(1S,2S-Cyclohexandiamin)],
- dem Komplex [Pt(II)$Cl_2$(1R,2R-Cyclohexandiamin)],
- dem Komplex [Pt(II)$Cl_2$(1R,2S-Cyclohexandiamin)], und
- dem Komplex [Pt(IV)Oxalato-trans-$Cl_2$(1R,2R-cyclo hexandiamin)],

und die Komplexe C2 ausgewählt sind aus

- Oxalplatin [Pt(II)Oxalato(1R,2R-cyclohexandiamin)],
- dem Komplex [Pt(II)Oxalato(R,S-cyclohexandiamin)],
- dem Komplex [Pt(II)$(NO_3)_2$ (1R,2R-Cyclohexandiamin)],
- dem Komplex [Pt(II)$(NO_3)_2$ (1S,2S-Cyclohexandiamin)],
- dem Komplex [Pt(II)$(NO_3)_2$ (1R,2S-Cyclohexandiamin)],
- Carboplatin [Pt (II)cis-Diammin(1,1-cyclobutandicarboxylat)], und

- dem Komplex [Pt(IV)Oxalato-trans-$Cl_2$-(1R,2Rcyclohexandiamin)].

**7.** Pharmazeutische Zusammensetzung, die als Wirkstoff eine Verbindung gemäß einem der Ansprüche 1 bis 3 enthält.

## Claims

**1.** A compound of formula (I) or (II):

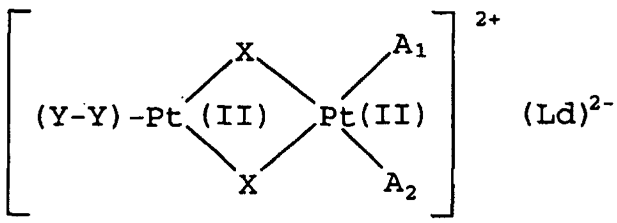
(I)

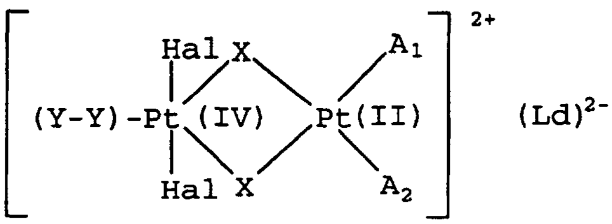
(II)

wherein:

- Y-Y is an amine bidentate ligand of the formula:
  - $NH_2$-$R_1$-$NH_2$ in which $R_1$ is a $C_2$-$C_6$ linear or branched alkylene group or an o-phenylene group;
  - or a group of the formula:

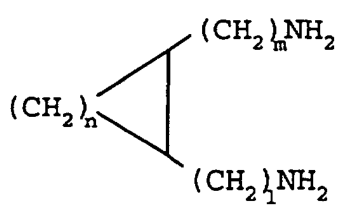

wherein

n = 2, 3, 4, 5;
m = 0 or 1 and
1 = 0 or 1;

- $A_1$ and $A_2$ represent either two identical ammine monodentate ligands, n-isopropylamine or ($C_3$-$C_7$) cycloalkylamine or they are bonded together thereby forming an amine bidentate ligand of the formula:
  - $NH_2$-$R_1$-$NH_2$ in which $R_1$ is a $C_2$-$C_6$ linear or branched alkylene group or an o-phenylene group;
  - or a group of the formula:

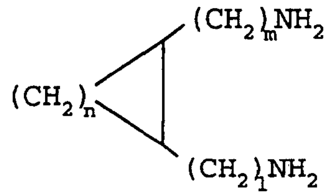

wherein

n = 2, 3, 4, 5;
m = 0 or 1 and
1 = 0 or 1;

- X represents a bridging ligand linking the two platinum atoms selected from Cl, Br, F and I;
- the dissociable Ld group represents:

  * either two monodentate B-B ligands selected from $NO_3^-$ or $RCOO^-$, in which R is a residue of gluconic or glucuronic acid;
  * or the bidentate B-B ligand selected from: $SO_4^{2-}$, $^-OOC\text{-}COO^-$,

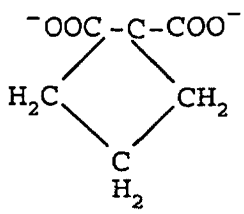 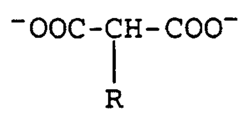

- wherein R is hydrogen, methyl, ethyl, phenyl or benzyl; and
- Hal is a halogen atom selected from the group consisting of Cl, Br, F and I.

**2.** The compound of formula (I) or (II) according to claim 1, **characterized by** the fact that :

- Y-Y represents a bidentate ligand selected from ethylenediamine, o-phenylenediamine, trimethylenediamine, 1,2-cyclohexanediamine and 2-(aminomethyl)cyclohexylamine;
- X is Cl;
- $A_1$ and $A_2$ represent either two ammine monodentate ligands or they are bonded together thereby forming a bidentate ligand selected from: ethylenediamine, o-phenylenediamine, trimethylenediamine, 1,2-cyclohexanediamine and 2-(aminomethyl)cyclohexylamine;
- Ld is:

  * either two monodentate B ligands selected from $NO_3^-$, or $RCOO^-$ in which R is a residue of gluconic and glucuronic acid;
  * or the bidentate ligand B-B selected from, $SO_4^{2-}$ or dicarboxylates consiting of oxolate, malonate 1,1-cyclobutanedicarboxylate; and

- Hal is Cl.

**3.** The compound of formula (I) or (II) according to claim 1, wherein said compound is selected from:

[(cis-diammine)(1R, 2R-diaminocyclohexane)-μ-dichloro-di-Pt(II)] oxalate;
[(cis-diammine)(1S,2S-diaminocyclohexane)-μ-dichloro-di-Pt(II)] oxalate,
[(cis-diammine)(R, S-diaminocyclohexane)-μ-dichloro-di-Pt(II)] oxalate,
[bis(1R, 2R-diaminocyclohexane)-μ-dichloro-di-Pt(II)] oxalate;
[(1R,2R-diaminocyclohexane) (1S, 2S-diaminocyclohexane)-μ-dichloro-di-Pt(II)] oxalate;

[(1R, 2R-diaminocyclohexane) (R,S-diaminocyclohexane-μ-dichloro-di-Pt(II)] oxalate;
[(cis-diammine)(1R, 2R-diaminocyclohexane)-μ-dichloro-di-Pt(II)] nitrate;
[(cis-diammine)(1S, 2S-diaminocyclohexane)-μ-dichloro-di-Pt(II)] nitrate;
[(cis-diammine) (R, S-diaminocyclohexane)-μ-dichloro-di-Pt(II) ] nitrate;
[bis (1R, 2R-diaminocyclohexane)-μ-dichloro-di-Pt(II)] nitrate;
[(1R, 2R-diaminocyclohexane)(1S, 2S-diaminocyclohexane)-μ-dichloro-di-Pt(II)] nitrate;
[(1R, 2R-diaminocyclohexane)(R, S-diaminocyclohexane)-μ -dichloro-di-Pt(II)] nitrate;
[bis (cis-diammine)-μ-dichloro-di-Pt(II)]1,1-cyclobutanedicarboxylate;
[(cis-diammine)(transdichloro-(1R, 2R-diaminocyclohexane)-μ-dichloro-Pt(II)-Pt(IV)] oxalate.

**4.** A process for the preparation of a compound of formula (I) or (II), **characterized in that** it consists of reacting, in an aqueous solution and in equimolar proportion, two mononuclear platinum complexes, one of which is a di-halogenated platinum complex C1 and the other a platinum complex C2 comprising either two monodentate B ligands or one bidentate B-B ligand as defined in claim 1 such that a bridge:

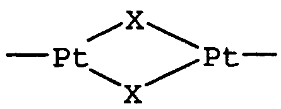

is formed between the two complexes, in which X is as defined in claim 1.

**5.** The process according to claim 4, which further consists of:

a) dissolving the mononuclear platinum complex C1 in distilled water by the application of heat in a water-bath;
b) dissolving, as in step a), the mononuclear platinum complex C2 in distilled water;
c) mixing the two solutions and heating the reaction mixture at 80°C;
d) allowing the reaction mixture to stand at room temperature; and
e) filtering and washing the needle- or scale-shaped pale yellow crystalline precipitate which forms in step d) with warm water and then with alcohol.

**6.** The process according to claim 5 complex C1 is selected from:

cisplatin: [Pt(II)cis $Cl_2$ $(NH_3)_2$];
[Pt(II)$Cl_2$ (1S, 2S-diaminocyclohexane)];
[Pt(II)-$Cl_2$ (1R,2R-diaminocyclohexane)];
[Pt(II)-$Cl_2$ (1R,2S-diaminocyclohexane));
[Pt(IV)-oxalate trans $Cl_2$ (1R, 2R-diaminocyclohexane)],

and the complex C2 is selected from :

oxaliplatin: [Pt(II)oxalate (1R, 2R-diaminocyclohexane)];
[Pt(II)oxalate(R, S-diaminocyclohexane)];
[Pt(II)$(NO_3)_2$(1R, 2R-diaminocyclohexane)];
[Pt(II)$(NO_3)_2$ (1S, 2S-diaminocyclohexane)];
[Pt(II)$(NO_3)_2$ (1R, 2S-diaminocyclohexane)] ;
carboplatin: [Pt(II)cis-diammine (1,1-cyclobutanedicarboxylate)];
[Pt(IV)-oxalate trans $Cl_2$ (1R, 2R-diaminocyclohexane)].

**7.** A pharmaceutical composition containing a compound according to any one of claims 1 to 3 as the active principle.

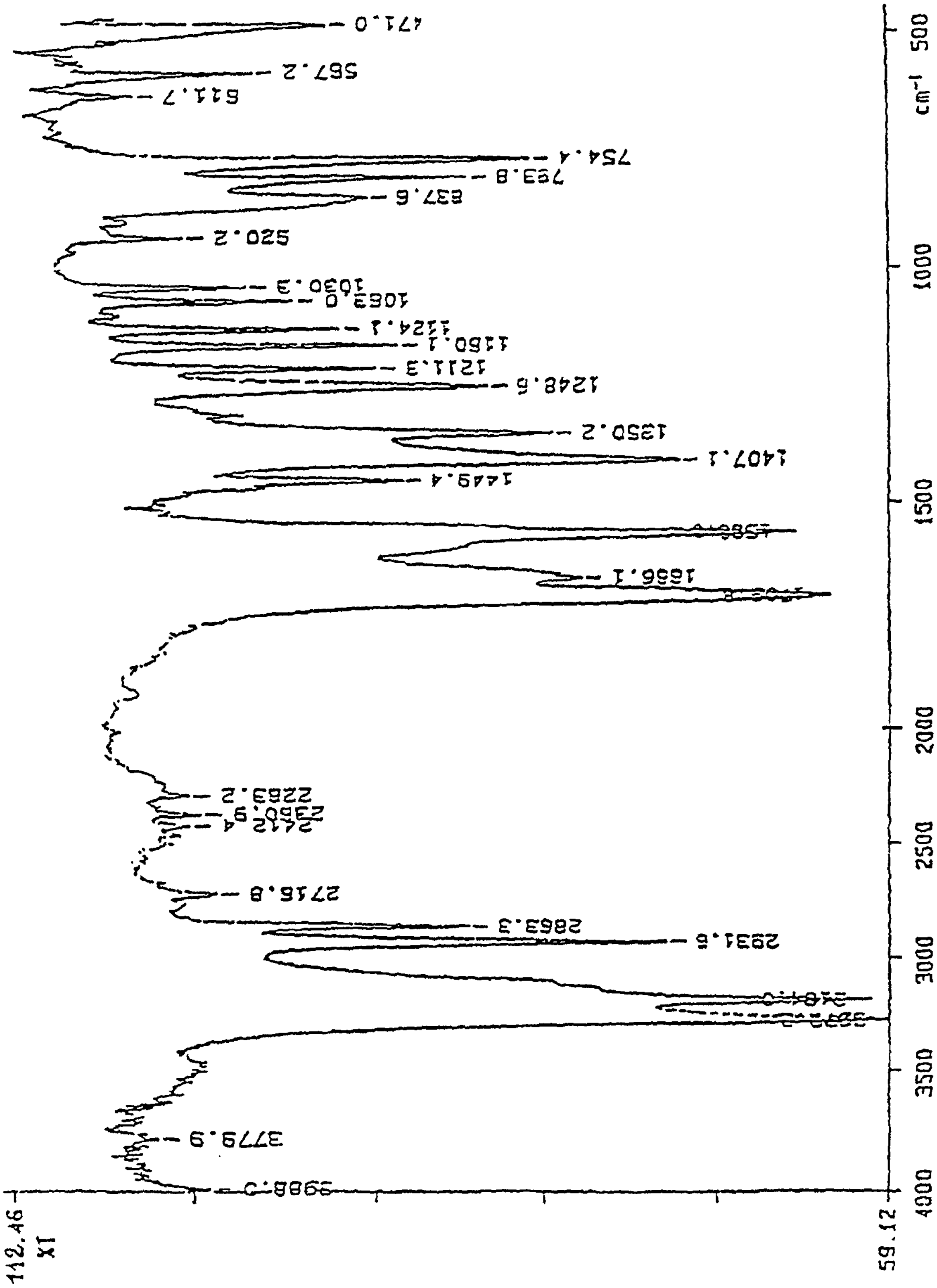
471.0
567.2
611.7
754.4
793.8
837.6
920.2
1030.3
1063.0
1124.1
1160.1
1211.3
1248.6
1350.2
1407.1
1449.4
1686.1
2263.2
2360.9
2412.4
2715.8
2863.3
2931.6
3779.9
3988.0
500
1000
1500
2000
2500
3000
3500
4000
cm-1
112.46
XT
59.12

FIG.1

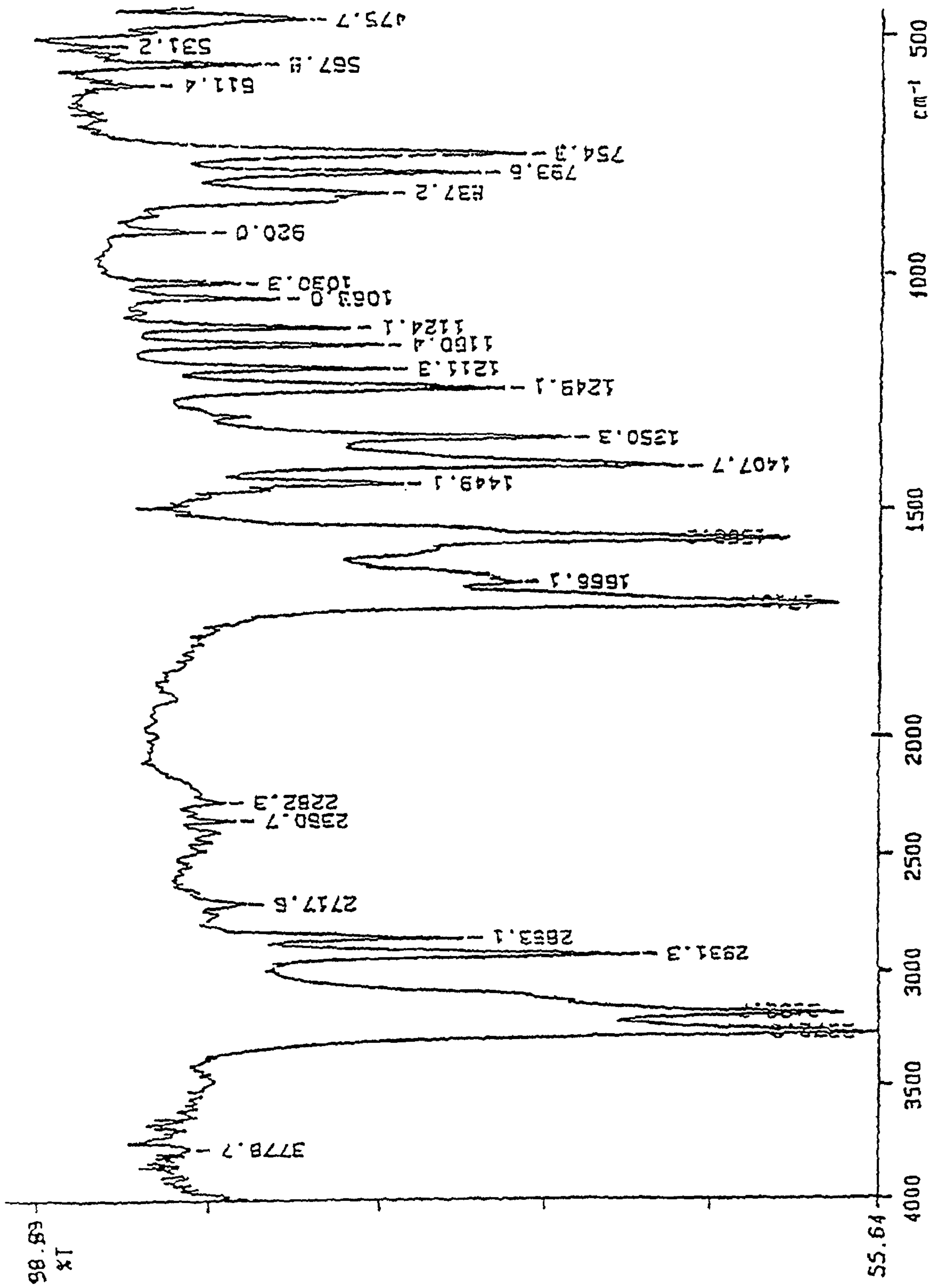
98.83
%T
55.64
cm-1
500
1000
1500
2000
2500
3000
3500
4000
475.7
531.2
567.8
611.4
754.3
793.6
837.2
920.0
1030.3
1063.0
1124.1
1160.4
1211.3
1249.1
1350.3
1407.7
1449.1
1666.1
2282.3
2380.7
2717.6
2853.1
2931.3
3778.7

FIG.2

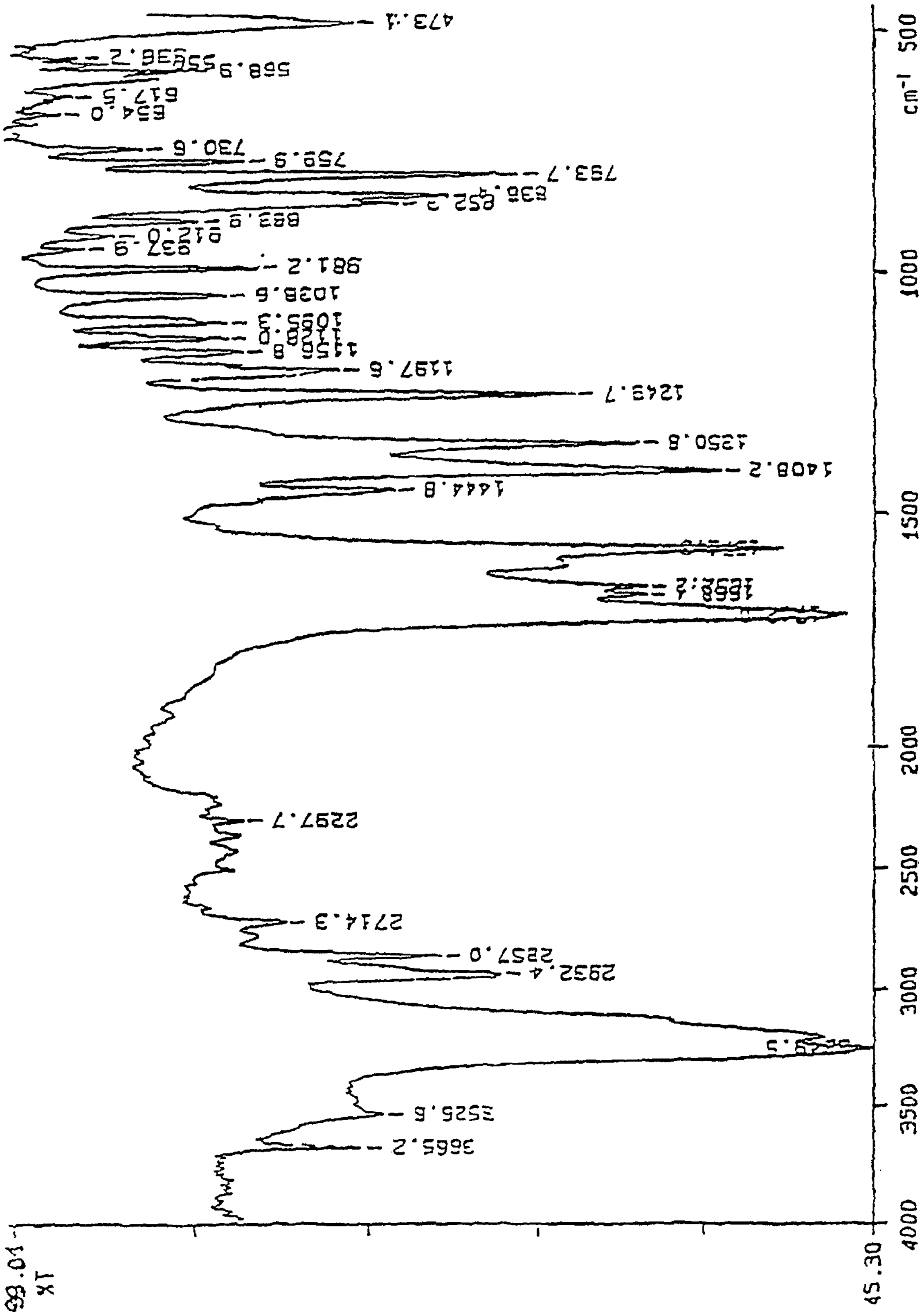
99.01
%T
45.30
cm-1
500
1000
1500
2000
2500
3000
3500
4000
473.1
538.2
568.9
617.5
654.0
730.6
759.9
793.7
838.4
852.2
883.9
912.0
937.9
981.2
1028.6
1085.3
1128.0
1156.8
1197.6
1243.7
1250.8
1408.2
1444.8
1652.2
1668.4
2297.7
2714.3
2857.0
2932.4
3525.8
3665.2

FIG.3

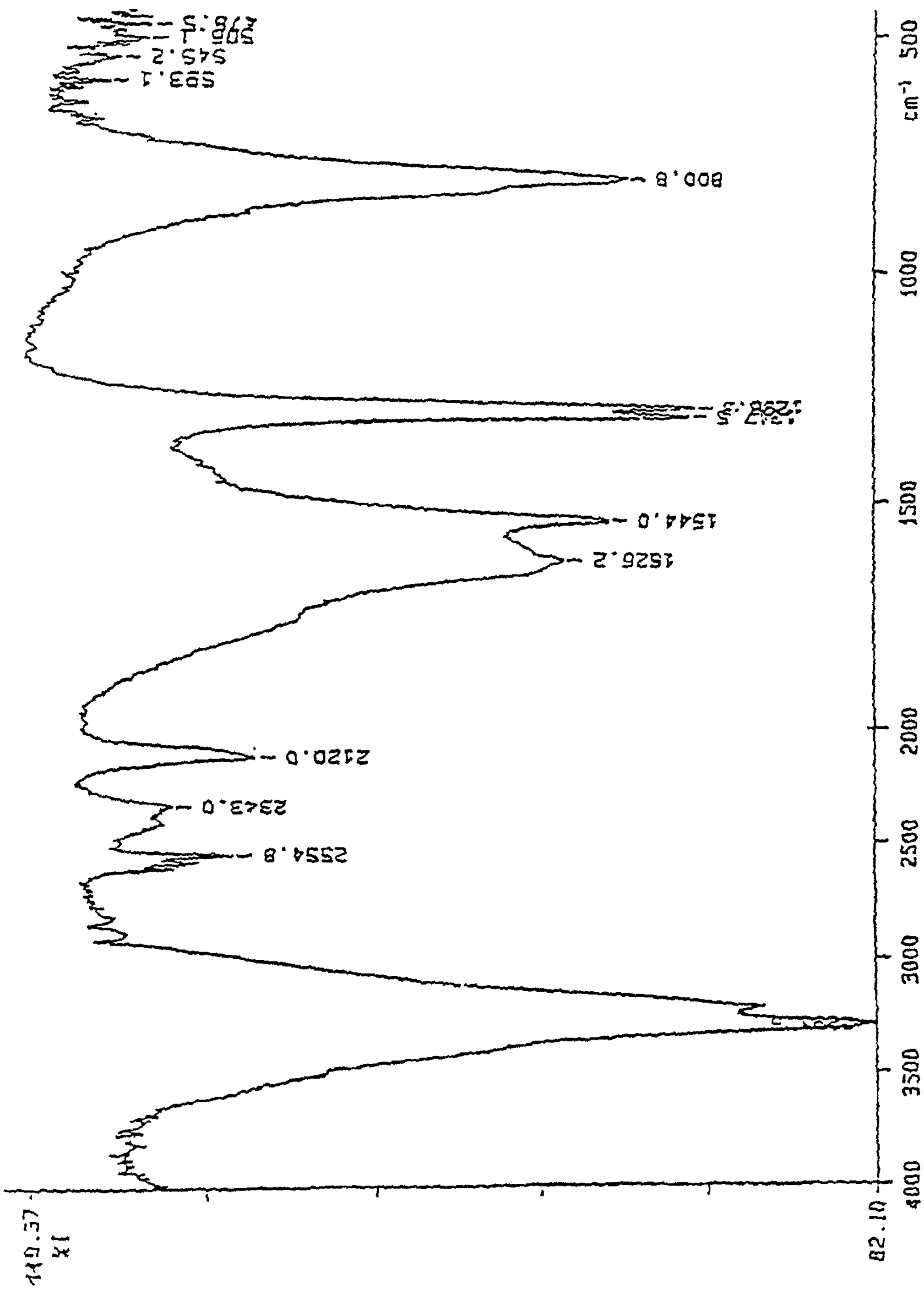

800.8
1544.0
2120.0
2343.0
2554.8
593.1
545.2
$cm^{-1}$
500
1000
1500
2000
2500
3000
3500
4000
82.10


FIG.4

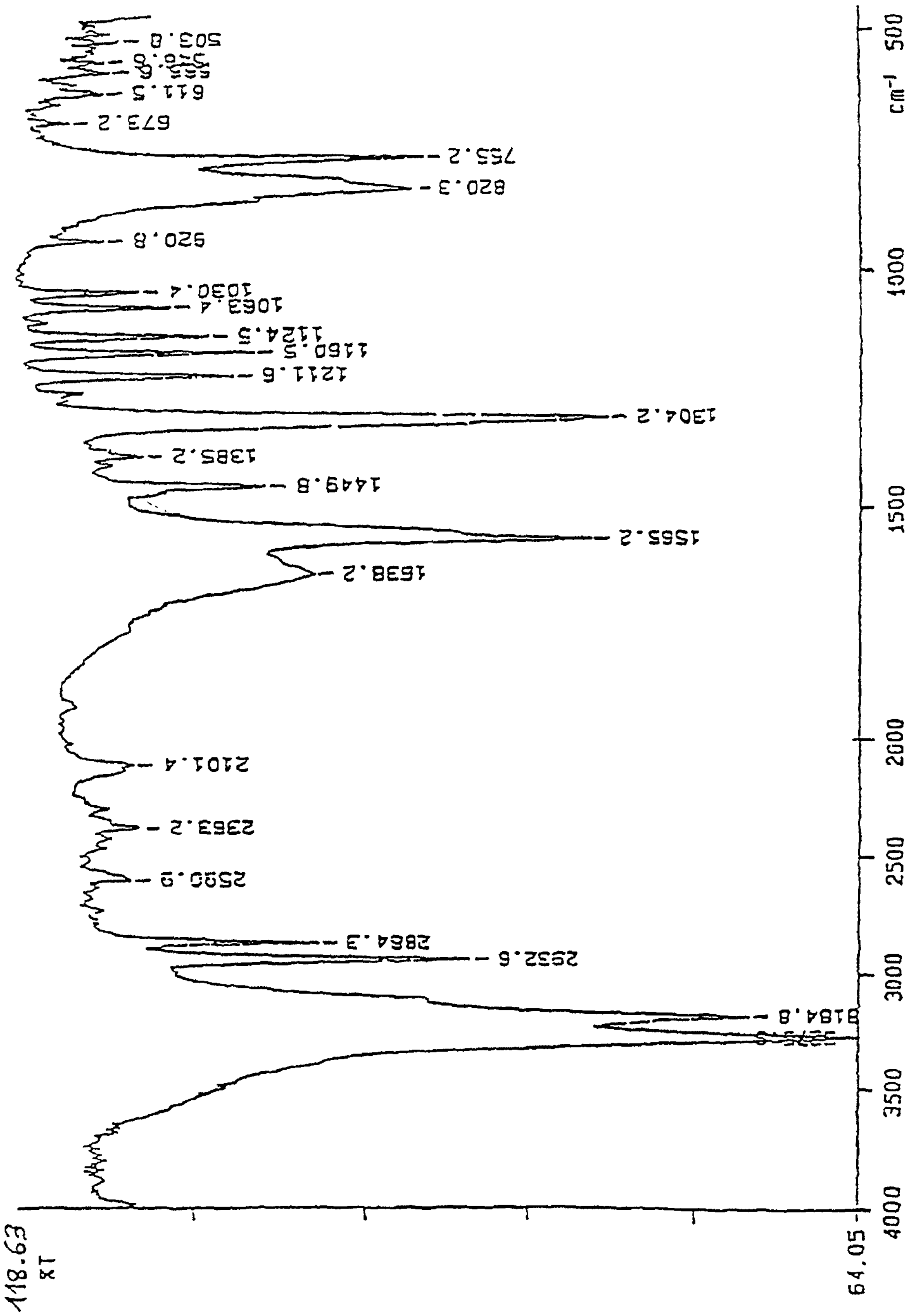
118.63
%T
64.05
4000
3500
3000
2500
2000
1500
1000
500
cm-1
3184.8
2932.6
2864.3
2520.9
2363.2
2101.4
1638.2
1565.2
1449.8
1385.2
1304.2
1211.6
1160.5
1124.5
1063.4
1030.4
920.8
820.3
755.2
673.2
611.5
503.8

FIG.5